# EUROPÄISCHE PATENTSCHRIFT

(11) **EP 1 415 605 B1**
(45) Veröffentlichungstag und Bekanntmachung des Hinweises auf die Patenterteilung: **13.10.2010**
(21) Anmeldenummer: 03012456.4
(22) Anmeldetag: 30.05.2003
(51) Int. Cl.: A61B 17/72, A61B 17/74

(54) **Knochenfixierungssystem**
Bone fixation system
Système de fixation d'os

(30) Priorität: 04.11.2002 EP 02024607
(43) Veröffentlichungstag der Anmeldung: 06.05.2004
(73) Patentinhaber: Zimmer GmbH, 8404 Winterthur (CH)
(72) Erfinder: Adam, Michael, 6402 Merlischachen (CH)
(74) Vertreter: Manitz, Finsterwald & Partner GbR

(56) Entgegenhaltungen:
- DE-A- 19 723 339
- US-B1- 6 331 179

## Beschreibung

Die Erfindung betrifft ein Knochenfixierungssystem mit wenigstens einem Nagel und zumindest einer durch eine im Nagel ausgebildete Querbohrung hindurchführbaren Schraube.

Derartige Systeme dienen insbesondere zur Reposition distaler Femurfrakturen, wie sie beispielsweise im "Manual der Osteosynthese", 3. Auflage, Springer-Verlag, Autoren: M. E. Müller, M. Allgöwer, R. Schneider, H. Willenegger, auf Seite 141 dargestellt sind. Bei dem Nagel handelt es sich insbesondere um einen Femurmarknagel, der vom Kniegelenk her eingesetzt wird und auf den eine Zielvorrichtung aufgesetzt werden kann, die es gestattet, mittels eines Bohrers die im Nagel ausgebildeten Querbohrungen in dem durch die Zielvorrichtung vorgegebenen, der Orientierung der Querbohrungen entsprechenden Winkel zu treffen und im Knochen Vorbohrungen für die anschließend einzusetzenden Schrauben auszubilden. Diese insbesondere in Form von Kondylenschrauben vorgesehenen Schrauben dienen dazu, den Marknagel bezüglich seiner axialen Richtung im Knochen zu fixieren.

Ein derartiges Fixierungssystem kommt auch dann zum Einsatz, wenn mittels der durch die Querbohrungen des Nagels gesteckten Schrauben Kondylenfragmente zum Restknochen hin fixiert werden sollen. Insbesondere dann, wenn sich die jeweilige Fraktur durch eine Mehrzahl von Kondylenfragmenten auszeichnet, stellt der Marknagel, der im Restknochen gehalten und mittels dortiger Verriegelungsschrauben fixiert ist, die einzige feste Bezugsbasis für die Fixierung der Kondylenfragmente dar. Beispiele für derartige Frakturen sind in dem vorstehend erwähnten "Manual der Osteosynthese" auf Seite 141 in den Abbildungen C1, C2 und C3 gezeigt.

Als problematisch kann sich bei derartigen Frakturen erweisen, dass die zur Fixierung der Kondylenfragmente dienenden, durch die Querbohrungen des Marknagels hindurchgeführten Knochenschrauben bezüglich ihrer eigenen Axialrichtung relativ zum Marknagel im Wesentlichen frei beweglich sind.

Aus der DE 197 23 339 C2 ist ein Implantat bekannt, das einen Markraumnagel sowie einen Schenkelhalsnagel umfasst und zur Fixierung von Frakturen im Schenkelhalsbereich des Femurknochens dient. Zur Festlegung des Schenkelhalsnagels in einer Durchstecköffnung des Markraumnagels ist ein den Schenkelhalsnagel im Bereich der Durchstecköffnung umgebender Klemmring vorgesehen, der mittels eines in axialer Richtung auf dem Schenkelhalsnagel verschiebbaren Spannelementes aufgeweitet werden kann.

Aufgabe der Erfindung ist es, ein Knochenfixierungssystem der eingangs genannten Art derart weiterzubilden, dass der Nagel auf möglichst einfache und sichere Weise mittels der Schraube zuverlässig und dauerhaft fixiert werden kann, wobei es insbesondere möglich sein soll, bei der Reposition distaler Femurfrakturen eine Mehrzahl von Kondylenfragmenten zuverlässig in ihrer korrekten Position zum Restknochen hin zu fixieren.

Die Lösung dieser Aufgabe erfolgt durch die Merkmale des Anspruchs 1 und insbesondere dadurch, dass eine Klemmeinrichtung vorgesehen ist, die wenigstens ein die Schraube zumindest bereichsweise umgebendes Aufspreizelement umfasst, das relativ zur Schraube axial verstellbar und während seiner Verstellbewegung zumindest im Bereich der Querbohrung aufspreizbar ist.

Erfindungsgemäß ist das Aufspreizelement gleichzeitig axial verstellbar und zumindest im Bereich der Querbohrung aufspreizbar. Hierdurch kann eine besonders einfach und zuverlässig betätigbare Klemmeinrichtung realisiert werden, die für eine sichere Fixierung der Schraube in der Bohrung des Nagels sorgt. Das Aufspreizelement ist hülsenförmig ausgebildet.

In einer besonders bevorzugten Ausführungsform der Erfindung ist das Aufspreizelement durch Zusammenwirken mit zumindest einer Querschnittserweiterung der Schraube aufspreizbar. Hierbei ist es die Schraube selbst, die automatisch für das Aufspreizen des Aufspreizelementes sorgt, wenn dieses während seiner Verstellbewegung axial relativ zur Schraube bewegt wird und dabei mit der Querschnittserweiterung der Schraube zusammenwirkt.

Besonders bevorzugt ist es, wenn die Querschnittserweiterung der Schraube in Form einer Auflauframpe für das Aufspreizelement vorgesehen ist. Hierdurch lässt sich ein besonders gleichmäßiges Aufspreizen des Aufspreizelementes realisieren.

Die Fixierung der Schraube im Nagel kann auf besonders einfache Weise vom Schraubenkopf aus gesteuert werden, wenn gemäß einem weiteren bevorzugten Ausführungsbeispiel der Erfindung das Aufspreizelement sich über eine axiale Länge der Schraube erstreckt, die von einem Bereich am oder in der Nähe des Schraubenkopfes bis in den Bereich der Querschnittserweiterung der Schraube reicht.

Eine besonders sichere Verankerung der Schraube im Nagel wird erreicht, wenn gemäß einer weiteren Ausführungsform der Erfindung das Aufspreizelement eine Mehrzahl von in Umfangsrichtung verteilt angeordneten Klemmelementen aufweist.

In einer bevorzugten Variante der Erfindung weist das Aufspreizelement eine Mehrzahl von lediglich durch Längsschlitze voneinander getrennten Klemmsegmenten auf. Hierdurch steht im Wesentlichen der gesamte Umfang des Aufspreizelementes oder zumindest ein wesentlicher Teil des Umfangs für das Verklemmen der Schraube im Nagel zur Verfügung. Eine exakte Ausrichtung der Schraube mit der Längsachse der im Nagel ausgebildeten Querbohrung ist hierdurch sichergestellt.

In einer weiteren Variante der Erfindung kann das Aufspreizelement wenigstens ein Paar von einander diametral gegenüberliegenden Klemmzungen aufweisen, die bevorzugt jeweils einen Winkelbereich abdecken, der kleiner ist als die Lücke zwischen den Klemmzungen.

In einer besonders bevorzugten Ausführung ist genau ein Paar von Klemmzungen vorgesehen, wobei vorzugsweise der von den Klemmzungen abgedeckte Winkelbereich derart klein ist, dass das Aufspreizelement von gabelartiger Gestalt ist. Es hat sich gezeigt, dass auch eine derartige, vergleichsweise geringfügige Abdeckung des Umfangs der Schraube durch das Aufspreizelement eine sichere Fixierung der Schraube in der Querbohrung des Nagels ermöglicht.

Ferner ist es bevorzugt, dass eine für das Aufspreizelement vorgesehene Querschnittserweiterung der Schraube von einem reduzierten Querschnitt auf den Normalquerschnitt der Schraube führt. Das Aufspreizen des Aufspreizelementes erfolgt somit durch den Übergang von dem Bereich mit reduziertem Querschnitt, in welchem das Aufspreizelement angeordnet ist, in den den Normalquerschnitt der Schraube aufweisenden Bereich, wobei unter Normalquerschnitt der Querschnitt einer herkömmlichen Schraube zu verstehen ist, die passgenau oder mit einem geringen radialen Spiel durch die Querbohrung des Nagels hindurchsteckbar ist.

Eine Schwächung der Schraube durch Querschnittsreduzierung lässt sich auf ein Minimum reduzieren, wenn gemäß einem besonders bevorzugten Ausführungsbeispiel der Erfindung der Querschnitt der Schraube ausschließlich in für die Klemmelemente des Aufspreizelementes vorgesehenen Bereichen reduziert ist. Besonders vorteilhaft ist in diesem Fall die vorstehend bereits erwähnte Ausgestaltung des Aufspreizelementes, wonach genau ein Paar von zusammen einen vergleichsweise kleinen Winkelbereich abdeckenden Klemmzungen vorgesehen und das Aufspreizelement insbesondere von gabelartiger Gestalt ist. Beispielsweise die Ausbildung von nut- oder rinnenartigen, auch als Keilbahnen bezeichneten Vertiefungen oder Ausnehmungen in der Schraube für die Klemmzungen ist dabei ausreichend.

Gemäß einer weiteren bevorzugten Ausführungsform ist die das Aufspreizelement umfassende Klemmeinrichtung mit der Schraube verschraubbar. Hierzu ist bevorzugt vorgesehen, dass die Schraube im Kopfbereich einen Gewindeabschnitt zum Verschrauben mit der Klemmeinrichtung aufweist.

Besonders bevorzugt ist es, wenn die Klemmeinrichtung derart ausgebildet ist, dass eine Schraubbewegung der Klemmeinrichtung in eine axiale Stellbewegung des Aufspreizelementes umsetzbar ist.

Während es prinzipiell möglich ist, die Klemmeinrichtung einteilig und damit lediglich aus dem Aufspreizelement bestehend auszubilden, wobei das Aufspreizelement durch Verschrauben mit der Schraube gleichzeitig axial verstellt wird, ist gemäß einer besonders bevorzugten Ausführungsform der Erfindung vorgesehen, dass die Klemmeinrichtung mehrteilig ausgebildet ist und zusätzlich zu dem über die Schraube schiebbaren Aufspreizelement ein mit der Schraube verschraubbares Verstellorgan umfasst, das mit dem Aufspreizelement axial fest gekoppelt oder koppelbar ist. Die Koppelung erfolgt insbesondere über eine Rast- oder Schnappverbindung.

Dabei ist es bevorzugt, wenn das Verstellorgan und das Aufspreizelement im axial fest gekoppelten Zustand gegeneinander verdrehbar sind. Hierdurch braucht lediglich das Verstellorgan der Klemmeinrichtung verdreht zu werden, während das Aufspreizelement eine reine Translationsbewegung in axialer Richtung ausführt.

In einem weiteren besonders bevorzugten Ausführungsbeispiel der Erfindung sind Begrenzungsmittel vorgesehen, die eine maximale Durchstecktiefe für die Schraube in der Querbohrung des Nagels festlegen. Hierdurch wird es auf einfache und zuverlässige Weise ermöglicht, mittels der Schraube z.B. ein Kondylenfragment an den Nagel und damit an den umgebenden Restknochen heranzuziehen und dabei je nach Anwendungsfall eine mehr oder weniger stark ausgeprägte Kompressionswirkung auszuüben, bevor die Schraube mittels der erfindungsgemäßen Klemmeinrichtung in der Querbohrung des Nagels in axialer Richtung festgesetzt wird. Hierbei wird außerdem in vorteilhafter Weise ausgenutzt, dass das Aufspreizelement gleichzeitig als Verdrehsicherung für die Schraube wirksam ist.

Des weiteren kann erfindungsgemäß vorgesehen sein, dass das Aufspreizelement durch Zusammenwirken mit einer Profilierung der Schraube gleichzeitig an mehreren in axialer Richtung aufeinander folgenden Stellen aufspreizbar ist.

Durch eine derartige Profilierung der Schraube kann erreicht werden, dass das Aufspreizelement über eine vergleichsweise große axiale Länge radial nach außen getrieben wird, wenn es relativ zur Schraube axial verstellt wird. Die Länge dieser Aufspreizstrecke kann durch die Länge der an der Schraube ausgebildeten Profilierung gezielt vorgegeben werden. Ein Vorteil dieser Ausführungsform besteht darin, dass eine größere Toleranz in axialer Richtung beim Festsetzen der Schraube im Nagel vorhanden ist. Es kann auf diese Weise die gesamte Querbohrung des Nagels zum Festsetzen der Schraube genutzt werden.

Das Aufspreizelement kann an seiner mit der Profilierung der Schraube zusammenwirkenden Innenseite eine Gegenprofilierung aufweisen.

Die Profilierung der Schraube und die Gegenprofilierung des Aufspreizelementes können komplementär zueinander ausgebildet sein.

Ferner kann vorgesehen sein, dass die Profilierung der Schraube und die Gegenprofilierung des Aufspreizelementes sich jeweils über eine axiale Länge erstrecken, die wenigstens 50 % der Länge des Aufspreizelementes beträgt.

Die Profilierung der Schraube kann von wenigstens einem sich in axialer Richtung wiederholenden Schraubenabschnitt mit variierendem Querschnitt gebildet sein.

Insbesondere kann die Profilierung der Schraube eine Mehrzahl von axial hintereinander angeordneten Auflauframpen umfassen.

In einem möglichen Ausführungsbeispiel ist die Profilierung der Schraube sägezahnartig ausgebildet.

In einer alternativen Ausgestaltung ist die Profilierung der Schraube wellenförmig ausgebildet.

Außerdem ist eine Schraube beschrieben, insbesondere eine Kondylenschraube, für ein Knochenfixierungssystem, wie es vorstehend erläutert wurde, wobei sich die Schraube dadurch auszeichnet, dass sie mit einer Klemmeinrichtung koppelbar ist, die wenigstens ein Aufspreizelement umfasst, das im gekoppelten Zustand die Schraube zumindest bereichsweise umgibt und relativ zur Schraube axial verstellbar ist, wobei die Schraube zumindest eine Querschnittserweiterung aufweist, mittels welcher das Aufspreizelement während seiner Verstellbewegung aufspreizbar ist.

Des Weiteren ist eine Klemmeinrichtung für eine Schraube eines erfindungsgemäßen Knochenfixierungssystems beschrieben, wie es vorstehend erläutert wurde, wobei sich die Klemmeinrichtung durch wenigstens ein die Schraube zumindest bereichsweise umgebendes Aufspreizelement auszeichnet, das relativ zur Schraube axial verstellbar und während seiner Verstellbewegung durch Zusammenwirken mit der Schraube aufspreizbar ist.

Weitere bevorzugte Ausführungsformen der Erfindung sind in den abhängigen Ansprüchen, der Beschreibung sowie der Zeichnung angegeben.

Die Erfindung wird im Folgenden beispielhaft unter Bezugnahme auf die Zeichnung beschrieben. Es zeigen:
- Fig. 1: ein Knochenfixierungssystem gemäß einer ersten Ausführungsform der Erfindung,
- Fig. 2: eine Schraube des Systems von Fig. 1,
- Fig. 3: ein Aufspreizelement des Systems von Fig. 1,
- Fig. 4: verschiedene Ansichten eines Verstellorgans des Systems von Fig. 1,
- Fig. 5: eine Schraube, ein Aufspreizelement und ein Verstellorgan des Systems von Fig. 1 im zusammengesetzten Zustand,
- Fig. 6: verschiedene Ansichten eines Nagels des Systems von Fig. 1,
- Fig. 7: teilweise ein Knochenfixierungssystem gemäß einer zweiten Ausführungsform der Erfindung,
- Fig. 8: verschiedene Ansichten eines Nagels des Systems von Fig. 7,
- Fig. 9: eine Schraube, ein Aufspreizelement und ein Verstellorgan des Systems von Fig. 7 im zusammengesetzten Zustand,
- Fig. 10: ein Knochenfixierungssystem gemäß einer dritten Ausführungsform der Erfindung,
- Fig. 11: verschiedene Ansichten einer Schraube des Systems von Fig. 10,
- Fig. 12: verschiedene Ansichten eines Aufspreizelementes des Systems von Fig. 10,
- Fig. 13: eine Schraube, ein Aufspreizelement und ein Verstellorgan des Systems von Fig. 10 im zusammengesetzten Zustand,
- Fig. 14: eine Schraube, eine Aufspreizelement und ein Verstellorgan eines Knochenfixierungssystems gemäß einer vierten Ausführungsform der Erfindung,
- Fig. 15: die Teile der Fig. 14 im zusammengesetzten Zustand,
- Fig. 16: eine Schraube, ein Aufspreizelement und ein Verstellorgan eines Knochenfixierungssystems gemäß einer fünften Ausführungsform der Erfindung, und
- Fig. 17: die Teile der Fig. 16 im zusammengesetzten Zustand.

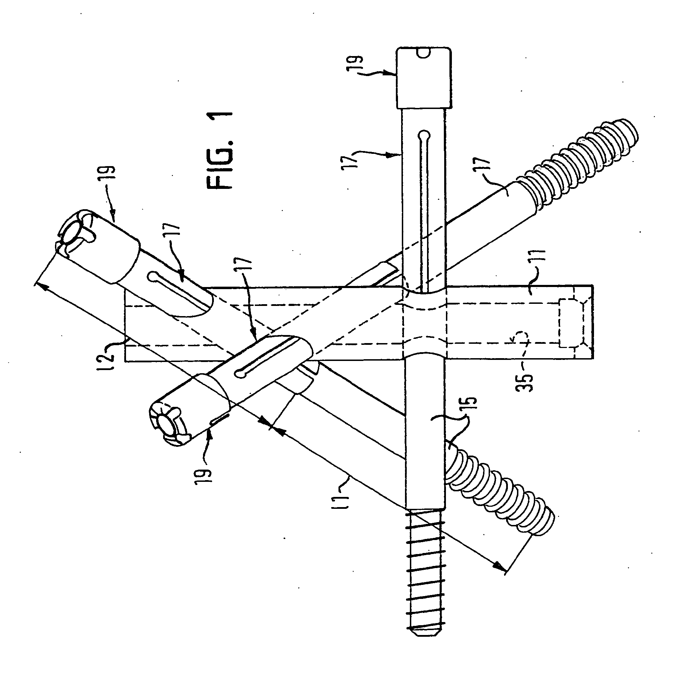

Das in den Fig. 1 bis 6 dargestellte Knochenfixierungssystem gemäß der ersten Ausführungsform der Erfindung umfasst in der Darstellung der Fig. 1 einen mit einer Längsbohrung 35 versehenen Femurmarknagel 11, der mit drei Querbohrungen versehen ist, durch welche Kondylenschrauben 15 gesteckt sind, die jeweils mittels einer nachstehend näher erläuterten Klemmeinrichtung 17, 19 in der Querbohrung des Nagels 11 festgesetzt und damit bezüglich ihrer Axialrichtung fixiert sind.

Sowohl der Nagel 11 als auch die Schrauben 15 gehören zu einem Satz von Teilen und werden für den jeweiligen Anwendungsfall passend ausgewählt. Insbesondere können sowohl die Gesamtlänge 11+12 der Schrauben 15 als auch die Länge 12 der Klemmeinrichtung 17, 19 sowie die Restlänge 11 der Schraube 15 verschiedene Werte annehmen.

Die Schrauben 15 sind jeweils im Bereich ihres freien Endes mit einem Gewindeabschnitt versehen, der in Fig. 1 lediglich angedeutet ist.

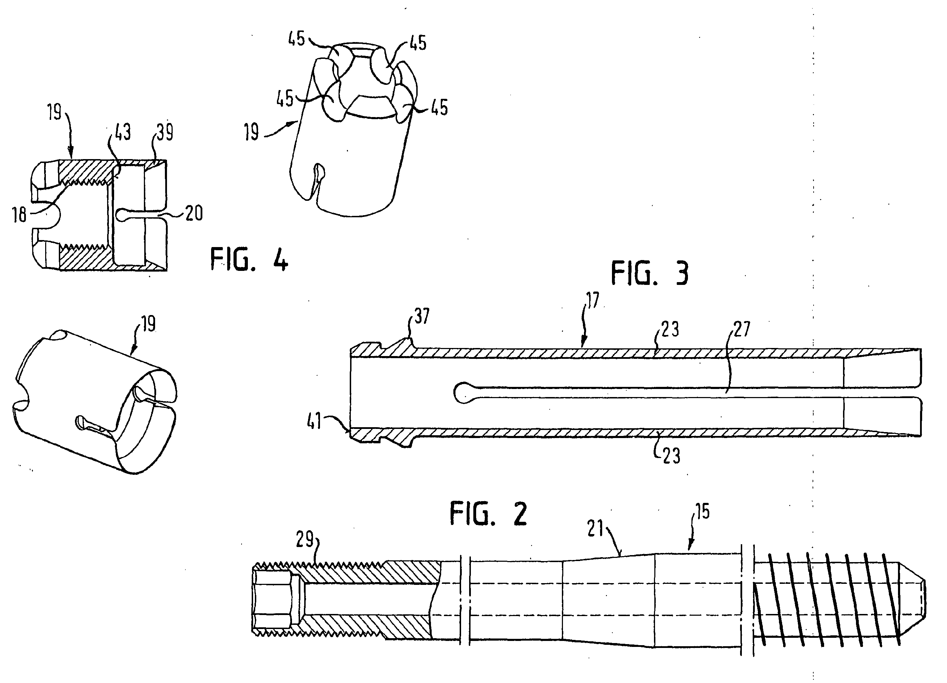

Von herkömmlichen Schrauben unterscheiden sich die erfindungsgemäßen Kondylenschrauben 15 gemäß Fig. 2 insbesondere durch einen im Kopfbereich ausgebildeten Außengewindeabschnitt 29 sowie eine in dem dargestellten Ausführungsbeispiel in Form einer Auflauframpe 21 vorgesehene Querschnittserweiterung. Vom Kopfbereich aus gesehen vor der Rampe 21 ist der Querschnitt der Schraube 15 über den gesamten Umfangsbereich reduziert, so dass die Schraube 15 im Bereich der Querschnittserweiterung 21 konusförmig ausgebildet ist. Die Auflauframpe 21 führt auf den Normalquerschnitt einer herkömmlichen Kondylenschraube.

Das Aufspreizelement 17 ist gemäß Fig. 3 hülsenförmig ausgebildet und weist zwei einander diametral gegenüberliegende Längsschlitze 27 auf, wodurch die Spreizhülse 17 in zwei Klemmsegmente oder Biegefedern 23 geteilt wird. Bei konstantem Außendurchmesser nimmt die Wandstärke der Aufspreizhülse 17 zu ihrem freien Ende hin entsprechend der Querschnittszunahme der Schraube 15 an der Auflauframpe 21 ab, wodurch sich die freie innere Querschnittsfläche der Aufspreizhülse 17 im Endbereich konusförmig erweitert.

Im Kopfbereich der Aufspreizhülse 17 sind Rastmittel 37 ausgebildet, über welche die Aufspreizhülse 17 axial fest mit dem in Fig. 4 dargestellten, in Form einer Stellschraube vorgesehenen Verstellorgan 19 gekoppelt werden kann, das mit entsprechenden Rastmitteln 39 versehen ist.

Der Koppelungsbereich der Stellschraube 19 ist mit Längsschlitzen 20 versehen, die beim Aufstecken der Stellschraube 19 auf den Kopfbereich der Aufspreizhülse 17 ein elastisches Aufweiten des Kopplungsbereiches der Stellschraube 19 gestatten.

Die Stellschraube 19 ist ferner mit einem Innengewindeabschnitt 18 versehen, über den die Stellschraube 19 mit dem Außengewindeabschnitt 29 der Kondylenschraube 15 verschraubbar ist.

Des Weiteren weist die Stellschraube 19 einen zentralen Durchgang auf, über den der Kopf der Kondylenschraube 15 bei aufgeschraubter Stellschraube 19 für ein Innensechskantwerkzeug zugänglich ist. Der stirnseitige Randbereich der Stellschraube 19 ist mit vier in Umfangsrichtung gleichmäßig beabstandeten Vertiefungen 45 versehen, an denen ein Werkzeug zum Drehen der Stellschraube 19 ansetzbar ist.

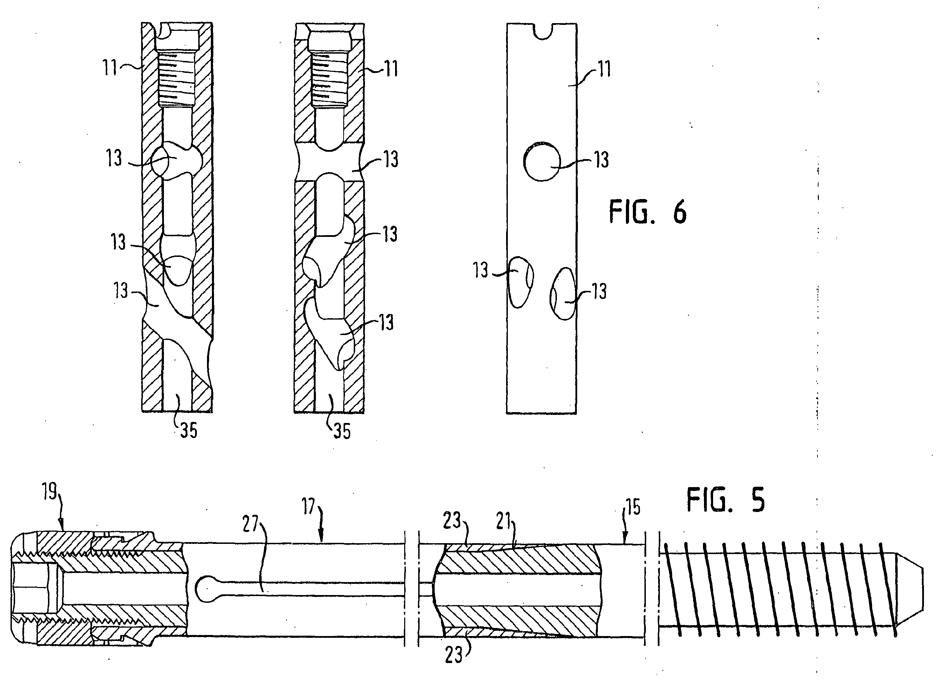

Fig. 5 zeigt den zusammengesetzten Zustand bei noch nicht aufgespreizter Hülse 17. Die Querschnittsreduzierung der Kondylenschraube 15 und die Wandstärke der Aufspreizhülse 17 sind einschließlich des Übergangsbereiches 21 derart aufeinander abgestimmt, dass mit Ausnahme des Kopfbereiches die Anordnung aus Kondylenschraube 15 und aufgeschobener Aufspreizhülse 17 einen konstanten Außendurchmesser aufweist.

Die mit der Klemmeinrichtung 17, 19 versehene Kondylenschraube 15 kann durch eine der im Nagel 11 ausgebildeten Querbohrungen 13 (vgl. Fig. 6) hindurchgeführt werden. Die Durchstecktiefe der Schraube 15 in der Querbohrung 13 des Nagels 11 ist durch die axiale Position der Auflauframpe 21 bestimmt, d.h. die Schraube 15 wird so weit durch die Querbohrung 13 hindurchgeführt, bis die Auflauframpe 21 und damit der Endbereich der Aufspreizhülse 17 im Bereich der Querbohrung 13 liegen.

Durch Verdrehen der Stellschraube 19 relativ zur Kondylenschraube 15 kann dann die Aufspreizhülse 17 in axialer Richtung bewegt werden, wobei (vgl. Fig. 3 und 4) die Stellschraube 20 mit einer Druckfläche 43 die Stirnseite 41 des Aufspreizelementes 17 beaufschlagt.

Durch die im Kopplungsbereich rotationssymmetrische Ausgestaltung der Stellschraube 19 und der Aufspreizhülse 17 dreht sich die Aufspreizhülse 17 beim Verdrehen der Stellschraube 19 nicht mit, d.h. die Schraubbewegung der Stellschraube 19 wird in eine reine axiale Translationsbewegung der Aufspreizhülse 17 umgesetzt.

Während der axialen Stellbewegung der Aufspreizhülse 17 werden die auf der Rampe 21 auflaufenden Klemmsegmente 23 radial auseinander gedrückt, wodurch die Hülse 17 aufgespreizt wird. Dabei kann sich das verjüngte, gewissermaßen spitz zulaufende freie Ende der Aufspreizhülse 17 sicher in den vorhandenen Zwischenraum zwischen der Kondylenschraube 15 und der die jeweilige Querbohrung 13 des Nagels 11 begrenzenden Innenwand schieben, wodurch eine stabile und dauerhafte Verspannung der Kondylenschraube 15 in der Querbohrung 13 des Nagels 11 erzielt und somit die Kondylenschraube 15 sowohl gegen axiale Bewegungen als auch gegen ein Verdrehen relativ zum Nagel 11 gesichert ist.

Zum Lösen der Klemmung wird die Stellschraube 19 in entgegengesetzter Richtung gedreht, wobei durch die Rast- oder Schnappkopplung zwischen der Stellschraube 19 und der Spreizhülse 17 eine sichere axiale Mitnahme der Spreizhülse 17 gewährleistet ist.

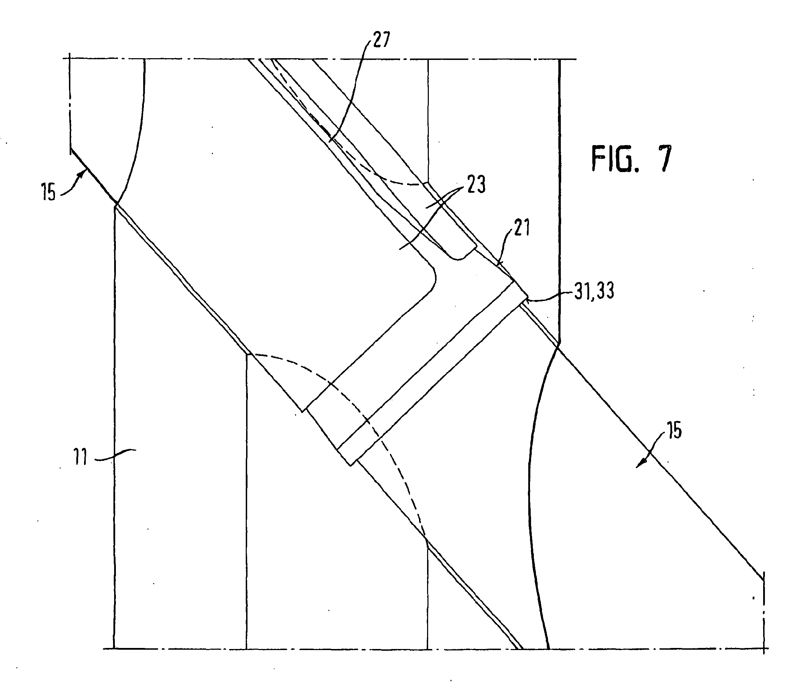

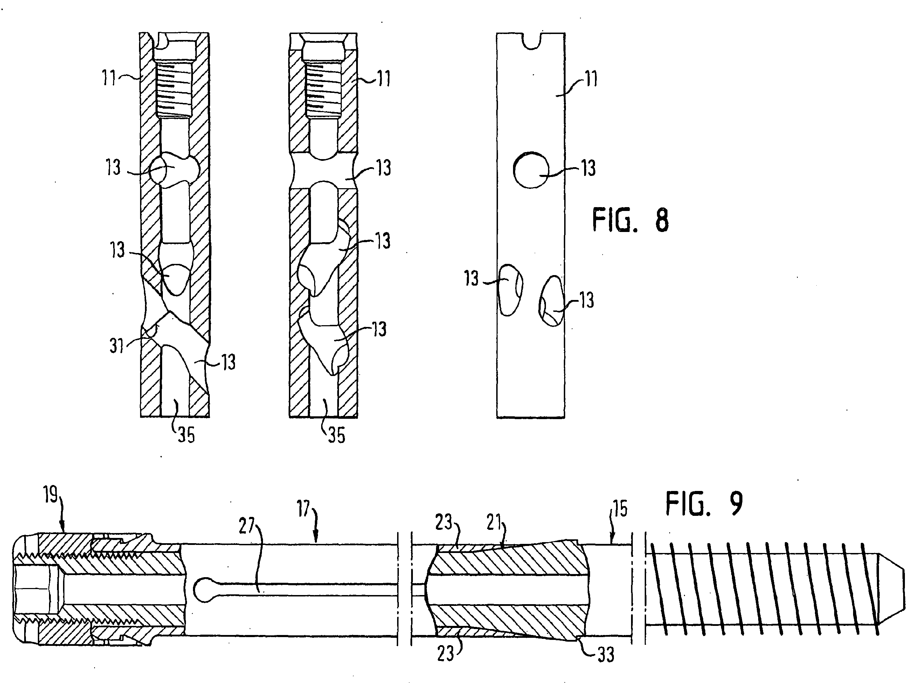

Die in den Fig. 7 bis 9 dargestellte zweite Ausführungsform der Erfindung unterscheidet sich von der ersten Ausführungsform im Wesentlichen dadurch, dass die Kondylenschraube 15 am vom Schraubenkopf aus gesehen hinteren Endbereich der Auflauframpe 21 mit einer Ringschulter 33 versehen ist, die mit einem in der betreffenden Querbohrung 13 des Nagels 11 ausgebildeten Ringanschlag 31 (vgl. insbesondere Fig. 8) zusammenwirkt, wenn die Kondylenschraube 15 in die Querbohrung 13 eingeführt wird.

Durch diese Anschlag- oder Begrenzungsmittel 31, 33 wird die Durchstecktiefe der Kondylenschraube 15 in der Querbohrung 13 begrenzt. Mittels der Schraube 15 können so Knochenfragmente an den Restknochen herangezogen werden, in den der Nagel 11 eingeschlagen ist.

Derartige Anschlag- oder Begrenzungsmittel 31 können in jeder Querbohrung 13 des Nagels 11 vorgesehen sein, wobei es aber auch möglich ist, dass eine oder mehrere Querbohrungen 13 keinen derartigen Anschlag für die Kondylenschraube 15 aufweisen.

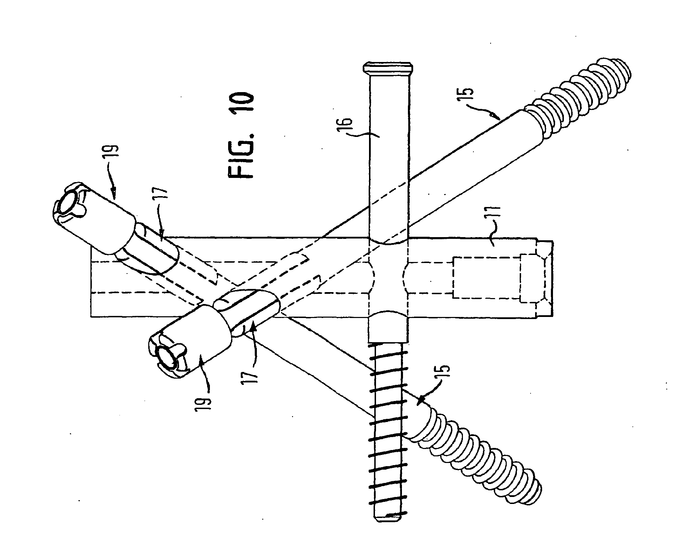

Die in den Fig. 10 bis 13 dargestellte dritte Ausführungsform der Erfindung unterscheidet sich von den zuvor erläuterten Ausführungsformen der Erfindung insbesondere durch die Ausgestaltung der Spreizhülse 17 und der für die Spreizhülse 17 vorgesehenen Querschnittsreduzierung der Kondylenschraube 15 in dem der Auflauframpe 21 vorgelagerten Bereich.

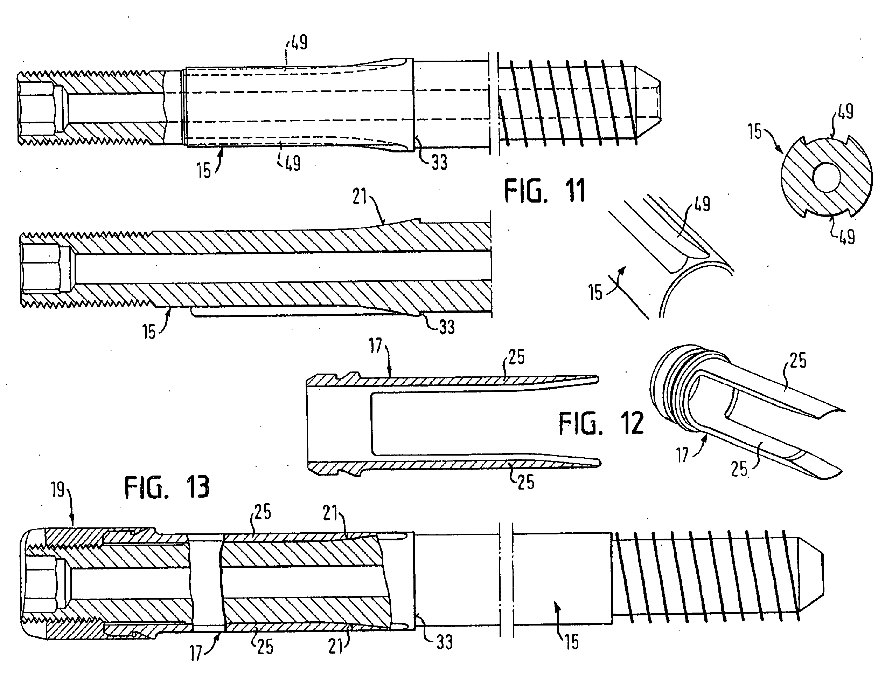

Die Spreizhülse 17 weist lediglich ein Paar von einander diametral gegenüberliegenden Klemmzungen 25 auf, die jeweils einen Winkelbereich abdecken, der kleiner ist als die Lücken zwischen den beiden Klemmzungen 25, so dass das Aufspreizelement 17 insgesamt von etwa gabelartiger Gestalt ist (vgl. insbesondere Fig. 12).

Zur Aufnahme der Klemmzungen 25 des Aufspreizelementes 17 ist die Kondylenschraube 15 mit rinnen- oder nutartigen Ausnehmungen 49 versehen, die auch als Keilbahnen bezeichnet werden. In ihren vom Schraubenkopf entfernten Endbereichen nimmt die Tiefe der Keilbahnen 49 allmählich ab, wodurch die Auflauframpen 21 für die Klemmzungen 25 des Aufspreizelementes 17 gebildet werden.

Durch diese auf den Bereich der Klemmzungen 25 beschränkte Querschnittsreduzierung der Kondylenschraube 15 wird deren Schwächung auf ein Minimum reduziert.

Die in Verbindung mit der zweiten Ausführungsform der Erfindung gemäß Fig. 7 bis 9 erläuterten Anschlag- oder Begrenzungsmittel 33 können auch bei den Kondylenschrauben 15 der dritten Ausführungsform vorgesehen sein, wie Fig. 11 und 13 zeigen. Zwingend ist dies jedoch nicht, d.h. bei der Ringschulter 33 einerseits und den Keilbahnen 49 andererseits handelt es sich um grundsätzlich unabhängig voneinander realisierbare Merkmale der erfindungsgemäßen Kondylenschrauben 15.

Fig. 10 zeigt am Beispiel der senkrecht zum Nagel 11 verlaufenden Schraube 16, dass nicht in jedem Anwendungsfall alle Schrauben mit einer erfindungsgemäßen Klemmeinrichtung versehen sein müssen. Vielmehr kann - und dies gilt für alle erfindungsgemäßen Ausführungsformen - auch ein "gemischtes" Knochenfixierungssystem zum Einsatz kommen, bei dem sowohl herkömmliche Schrauben 16 als auch die erfindungsgemäßen Klemmschrauben 15 verwendet werden.

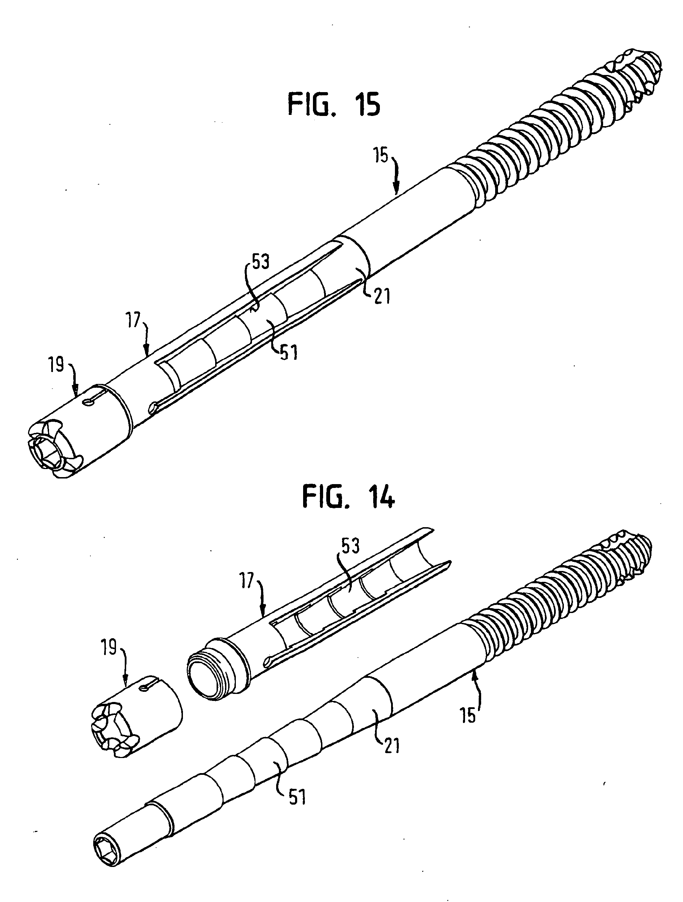

Die beiden in den Fig. 14 bis 17 dargestellten Ausführungsformen unterscheiden sich von den zuvor erläuterten Ausführungsformen insbesondere jeweils dadurch, dass die Schraube zusätzlich zu der mit den freien Endbereichen der Spreizhülse 17 zusammenwirkenden Auflauframpe 21 mit einer bezüglich der Schraubenlängsachse rotationssymmetrischen Oberflächenprofilierung 51 versehen ist.

Die Profilierung 51 ist - in Verstellrichtung der Spreizhülse 17 gesehender Auflauframpe 21 vorgelagert und erstreckt sich über eine wesentliche axiale Länge der Schraube.

Die Innenseite der hier lediglich aus Gründen der Veranschaulichung aufgebrochen dargestellten, hülsenförmig ausgebildeten und mit Längsschlitzen versehenen Spreizhülse 17 ist mit einer zu der Profilierung 51 der Schraube 15 komplementären Gegenprofilierung 53 versehen. Bei nicht aufgespreizter Spreizhülse 17 ergänzen sich die Spreizhülse 17 und die Schraube 15 zu einem zylindrischen Ganzen mit in axialer Richtung konstantem Querschnitt, der gleich dem Schraubenquerschnitt im Anschluss an die Auflauframpe 21 ist.

Wird die Spreizhülse 17 zum Festsetzen der Schraube in der Querbohrung eines hier nicht dargestellten Nagels mittels des Verstellorgans 19 in axialer Richtung verschoben, so wird die Spreizhülse 17 nicht nur im Bereich ihrer freien Enden durch die Auflauframpe 21, sondern außerdem durch das Zusammenwirken von Profilierung 51 der Schraube 15 und Gegenprofilierung 53 der Spreizhülse 17 aufgespreizt. Eine radiale Erweiterung der Spreizhülse 17 in dem Bereich zwischen ihren freien Enden und ihrem von dem Verstellorgan 19 beaufschlagten Kopfbereich erfolgt also nicht lediglich aufgrund einer der Spreizhülse 17 inne wohnenden Steifigkeit, sondern die Spreizhülse 17 wird aktiv durch die Profilierung 51 der Schraube 15 an mehreren in axialer Richtung beabstandeten Bereichen aufgeweitet. Folglich kann über einen wesentlichen axialen Bereich der Spreizhülse 17 eine Klemmkraft ausgeübt werden, was es ermöglicht, die gesamte Querbohrung des Marknagels zum Festsetzen der Schraube 15 zu nutzen.

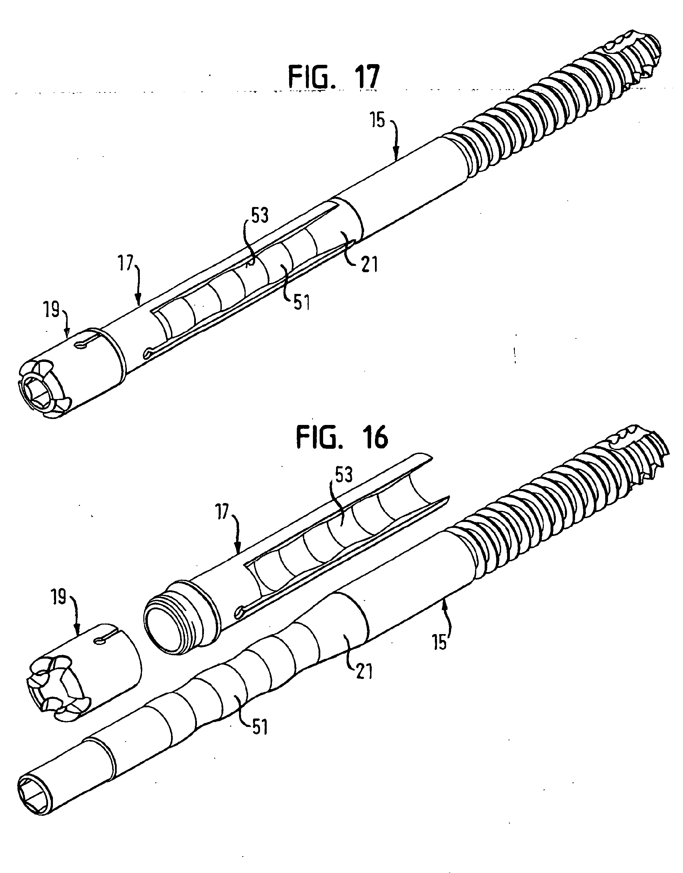

In der Ausführungsform der Fig. 14 und 15 sind Profilierung 51 und Gegenprofilierung 53 sägezahnartig ausgebildet, während in der Ausführungsform der Fig. 16 und 17 eine Wellenform für Profilierung 51 und Gegenprofilierung 53 vorgesehen ist. Jeder Sägezahn bzw. jeder Wellenberg der Schraubenprofilierung 51 bildet eine nach Art einer Auflauframpe wirkende Erhöhung, die einen entsprechenden Gegenabschnitt der Spreizhülsenprofilierung 53 radial nach außen zwingt, wenn die Spreizhülse 17 auf der Schranke 15 axial verschoben wird.

Die Sägezahnform und die Wellenform stellen konkrete Beispiele für ein vorteilhaftes Aufspreizen der Spreizhülse 17 über einen wesentlichen axialen Längenbereich ermöglichende Profilierungen und Gegenprofilierungen dar. Andere Profilierungsformen sind durchaus möglich, d.h. der nicht zwangsläufig, jedoch bevorzugt periodische axiale Verlauf der Querschnittsvariation kann von der Sägezahnform und der Wellenform abweichen und grundsätzlich beliebig geformte, abwechselnd - in radialer Richtung betrachtet - Höhen und Senken bildende Schraubenabschnitte umfassen.

### Bezugszeichenliste

- 11: Nagel
- 13: Querbohrung
- 15: Schraube
- 16: herkömmliche Schraube
- 17: Aufspreizelement
- 18: Innengewindeabschnitt des Verstellorgans
- 19: Verstellorgan, Stellschraube
- 20: Längsschlitz des Verstellorgans
- 21: Querschnittserweiterung, Auflauframpe
- 23: Klemmsegment
- 25: Klemmzunge
- 27: Längsschlitz
- 29: Gewindeabschnitt der Schraube
- 31: Ringanschlag des Nagels
- 33: Ringschulter der Schraube
- 35: Längsbohrung des Nagels
- 37: Rastmittel des Aufspreizelementes
- 39: Rastmittel des Verstellorgans
- 41: Stirnseite des Aufspreizelementes
- 43: Druckfläche des Verstellorgans
- 45: Vertiefung
- 49: Ausnehmung der Schraube
- 51: Profilierung
- 53: Gegenprofilierung

- 11: Restlänge der Schraube
- 12: Länge der Klemmeinrichtung

## Patentansprüche

1. Knochenfixierungssystem, insbesondere zur Reposition distaler Femurfrakturen, umfassend wenigstens einen Nagel (11), insbesondere einen Femurmarknagel, zumindest eine durch eine im Nagel (11) ausgebildete Querbohrung (13) hindurchführbare Schraube (15), insbesondere eine Kondylenschraube, und
eine Klemmeinrichtung, die wenigstens ein die Schraube (15) zumindes bereichsweise umgebendes Aufspreizelement (17) umfasst, das relativ zur Schraube (15) axial verstellbar und während seiner Verstellbewegung zumindest im Bereich der Querbohrung (13) aufspreizbar ist,
**dadurch gekennzeichnet,**
**dass** das Aufspreizelement (17) hülsenförmig ausgebildet ist.

2. Knochenfixierungssystem nach Anspruch 1,
**dadurch gekennzeichnet,**
**dass** das Aufspreizelement (17) durch Zusammenwirken mit zumindest einer Querschnittserweiterung (21) der Schraube (15) aufspreizbar ist.

3. Knochenfixierungssystem nach Anspruch 2,
**dadurch gekennzeichnet,**
**dass** die Querschnittserweiterung (21) der Schraube (15) in Form einer Auflauframpe für das Aufspreizelement (17) vorgesehen ist. und/oder die Schraube (15) im Bereich der Querschnittserweiterung (21) konusförmig ausgebildet ist.

4. Knochenfixierungssystem nach einem der vorhergehenden Ansprüche,
**dadurch gekennzeichnet,**
**dass** sich das Aufspreizelement (17) über eine axiale Länge der Schraube (15) erstreckt, die von einem Bereich am oder in der Nähe des Schraubenkopfes bis in den Bereich einer Querschnittserweiterung (21) der Schraube (15) reicht.

5. Knoehenfixierungssystem nach einem der vorhergehenden Ansprü-, che,
**dadurch gekennzeichnet,**
**dass** die Wandstärke des Aufspreizelementes (17) im Bereich des freien Endes in axialer Richtung sich verjüngt oder spitz zuläuft, und/ oder sich die freie innere Oucrschnittsftäche des Aufspreizelementes (17) im Bereich des freien Endes in axialer Richtung konusförmig erweitert.

6. Knochenfixerungssystem nach einem der vorhergehenden Ansprüche,
**dadurch gekennzeichnet,**
**dass** das Aufspreizelement (17) eine Mehrzahl von in Umfarigsrichtung verteilt angeordneten Klemmelementen (23, 25) aufweist.

7. Knochenfixierungssystem nach einem der vorhergehenden Ansprüche,
**dadurch gekennzeichnet,**
**dass** das Aufspreizelement (17) eine Mehrzahl von lediglich durch Längsschlitze (27) voneinander getrennten Klemmsegmenten (23) aufweist und/oder als eine geschlitzte Hülse ausgebildet ist.

8. Knochenfixierungssystem nach einem der Ansprüche 1 bis 6,
**dadurch gekennzeichnet,**
**dass** das Aufspreizelement (17) wenigstens ein Paar voneinander diametral gegenüberliegenden Klemmzungen (25) aufweist, die bevorzugt jeweils einen Umfanas-Winkelbereich abdecken, der kleiner ist als die Lücken zwischen den Klemmzungen (25).

9. Knochenfixierungssystem nach einem der vorhergehenden Ansprüche,
**dadurch gekennzeichnet,**
**dass** eine für das Aufspreizelement (17) vorgesehene Querschnittserweiterung (21) der Schraube (15) von einem reduzierten Querschnitt auf den Normalquerschnitt der Schraube (15) führt.

10. Knochenfixierungssystem nach einem der vorhergehenden Ansprüche,
**dadurch gekennzeichnet, dass**
der Querschnitt der Schraube (15) ausschließlich in für Klemmelemente (23, 25) des Aufspreizelementes (17) vorgesehenen Bereichen reduziert und hierzu bevorzugt mit nut- oder rinnenartigen Ausnehmungen (49) versehen ist.

11. Knochenfixierungssystem nach einem der vorhergehenden Ansprüche,
**dadurch gekennzeichnet,**
**dass,** die Klemmeinrichtung mit der Schraube (15) verschraubbar ist.

12. Knochenfixierungssystem nach einem der vorhergehenden Ansprüche,
**dadurch gekennzeichnet,**
**dass** die Schraube (15) im Kopfbereich einen Gewindeabschnitt (29) zum Verschrauben mit der Klemmeinrichtung aufweist.

13. Knochenfixierungssystem nach einem der vorhergehenden Ansprüche,
**dadurch gekennzeichnet,**
**dass** eine Schraubbewegüng der Klemmeinrichtung in eine axiale Stellbewegung des Aufspreizelementes (17) umsetzbar ist.

14. Knochenfixierungssystem nach einem der vorhergehenden Ansprüche,
**dadurch gekennzeichnet,**
**dass** die Klemmeinrichtung mehrteilig ausgebildet ist und zusätzlich zu dem über die Schraube (15) schiebbaren Aufspreizelement ein mit
der Schraube (15) verschraubbares Verstellorgan (1 9) umfasst, das mit dem Aufspreizelement (17) axial fest gekoppelt oder koppelbar ist, insbesondere über eine Rast- oder Schnappverbindung.

15. Knochennxierungssystem nach einem der vorhergehenden Ansprüche,
**dadurch gekennzeichnet,**
**dass** das Verstellorgan (19) und das Aufspreizelement (17) im axial fest gekoppelten Zustand gegeneinander verdrehbar sind.

16. Knochenfixierungssystem nach einem der vorhergehenden Ansprüche,
**dadurch gekennzeichnet,**
**dass** Begrenzungsmittel (31, 33) vorgesehen sind, die eine maximale Durchstecktiefe für die Schraube (15) in der Bohrung (13) des Nagels (11) festlegen, wobei vorzugsweise die Begrenzungsmittel einen Ringanschlag (31) an der die Bohrung (13) begrenzenden Innenwand des Nagels (11) sowie eine an der Schraube (15) ausgebildete Ringschulter (33) umfassen.

17. Knochenfixierungssystem nach einem der vorhergehenden Ansprüche,
**dadurch gekennzeichnet,**
**dass** das Aufspreizelement (17) durch Zusammenwirken mit einer Profilierung (51) der Schraube (15) gleichzeitig an mehreren in axialer Richtung aufeinander folgenden Stellen aufspreizbar ist.

18. Knochenfixierungssystem nach Anspruch 17,
**dadurch gekennzeichnet,**
**dass** das Aufspreizelement (17) an seiner mit der Profilierung (51) der Schraube (15) zusammenwirkenden Innenseite eine Gegenprofilierung (53) aufweist.

19. Knochenfixierungssystem nach Anspruch 18,
**dadurch gekennzeichnet,**
**dass** die Profilierung (51) der Schraube (15) und die Gegenprofilierung (53) des Aufspreizelementes (17) komplementär zueinander ausgebildet sind.

20. Knochennxierungssystem nach Anspruch 18 oder 19,
**dadurch gekennzeichnet,**
**dass** die Profilierung (15) der Schraube (551) und die Gegenprofilierung (53) des Aufspreizelementes (17) sich jeweils über eine axiale Länge erstrecken, die wenigstens 50% der Länge des Aufspreizelementes (17) beträgt.

21. Knochenfixierungssystem nach einem der Ansprüche 17 bis 20,
**dadurch gekennzeichnet,**
**dass** die Profilierung (51) der Schraube (15) von wenigstens einem sich in axialer Richtung wiederholenden Schraubenabschnitt mit variierendem Querschnitt gebildet ist.

22. Knochennxierungssystem nach einem der Ansprüche 17 bis 21,
**dadurch gekennzeichnet,**
**dass** die Profilierung (51) der Schraube (15) eine Mehrzahl von axial hintereinander angeordneten Auflauframpe umfasst.

23. Knochenfixierungssystem nach einem der Ansprüche 17 bis 22,
**dadurch gekennzeichnet,**
**dass** die Profilierung (51) der Schraube (15) sägezahnartig ausgebildet ist.

24. Knochenfixierungssystem nach einem der Ansprüche 17 bis 22,
**dadurch gekennzeichnet,**
**dass** die Profilierung (51) der Schraube (15) wellenförmig ausgebildet ist.

## Claims

1. A bone fixing system, in particular for the reposition of distal femoral fractures, comprising at least one nail (11), in particular a femoral medullary nail, at least one screw (15), in particular a condyle screw, which can be led through a transverse bore (13) formed in the nail (11) and a clamping device which includes at least one spreading member (17) which surrounds the screw (15) at least region-wise, is axially adjustable relative to the screw (15) and is spreadable at least in the region of the transverse bore (13) during its adjustment movement,
**characterized in that** the spreading member (17) is made in sleeve form.

2. A bone fixing system in accordance with claim 1, **characterized in that** the spreading member (17) is spreadable by cooperation with at least one cross-sectional expansion (21) of the screw (15).

3. A bone fixing system in accordance with claim 2, **characterized in that** the cross-sectional expansion (21) of the screw (15) is provided in the form of a run-up ramp for the spreading member (17); and/or in that the screw (15) is made in conical shape in the region of the cross-sectional expansion (21).

4. A bone fixing system in accordance with any one of the preceding claims, **characterized in that** the spreading member (17) extends over an axial length of the screw (15) which extends from a region at the screw head, or in the proximity of the screw head, up to and into the region of a cross-sectional expansion (21) of the screw (15).

5. A bone fixing system in accordance with any one of the preceding claims, **characterized in that** the wall thickness of the spreading member (17) tapers or converges to a tip in the axial direction in the region of the free end; and/or **in that** the free inner cross-sectional surface of the spreading member (17) expands in conical shape in the axial direction in the region of the free end.

6. A bone fixing system in accordance with any one of the preceding claims, **characterized in that** the spreading member (17) has a plurality of clamping elements (23, 25) arranged distributed in the peripheral direction.

7. A bone fixing system in accordance with any one of the preceding claims, **characterized in that** the spreading element (17) has a plurality of clamping elements (23) only separated from one another by elongate slots (27) and/or is made as a slit sleeve.

8. A bone fixing system in accordance with any one of claims 1 to 6, **characterized in that** the spreading member (17) has at least one pair of clamping tongues (25) which are disposed diametrically opposite one another and which preferably each cover a peripheral angular region which is smaller than the gaps between the clamping tongues (25).

9. A bone fixing system in accordance with any one of the preceding claims, **characterized in that** a cross-sectional expansion (21) of the screw (15) provided for the spreading member (17) leads from a reduced cross-section to the normal cross-section of the screw (15).

10. A bone fixing system in accordance with any one of the preceding claims, **characterized in that** the cross-section of the screw (15) is only reduced in regions provided for clamping elements (23, 25) of the spreading member (17) and is preferably provided for this purpose with groove-like or channel-like cut-outs (49).

11. A bone fixing system in accordance with any one of the preceding claims, **characterized in that** the clamping device can be screwed with the screw (15).

12. A bone fixing system in accordance with any one of the preceding claims, **characterized in that** the screw (15) has a thread section (29) in the head region for screwing to the clamping device.

13. A bone fixing system in accordance with any one of the preceding claims, **characterized in that** a screw movement of the clamping device can be converted into an axial adjusting movement of the spreading member (17).

14. A bone fixing system in accordance with any one of the preceding claims, **characterized in that** the clamping device is made in a plurality of parts and includes, in addition to the spreading member which can be pushed over the screw (15), a adjustment member (19) which can be screwed with the screw (15) and is or can be axially fixedly coupled to the spreading member (17), in particular via a latch connection or snap connection.

15. A bone fixing system in accordance with any one of the preceding claims, **characterized in that** the adjustment member (19) and the spreading member (17) can be rotated relative to one another in the axially fixedly coupled state.

16. A bone fixing system in accordance with any one of the preceding claims, **characterized in that** bounding means (31, 33) are provided which define a maximum penetration depth for the screw (15) in the bore (13) of the nail (11), with the bounding means preferably including a ring abutment (31) at the inner wall of the nail (11) bounding the bore (13) and a ring shoulder (33) formed at the screw (15).

17. A bone fixing system in accordance with any one of the preceding claims, **characterized in that** the spreading member (17) can be spread apart at a plurality of successive positions in the axial direction simultaneously by cooperating with a profile (51) of the screw (15).

18. A bone fixing system in accordance with claim 17, **characterized in that** the spreading member (17) has a counter-profile (53) at its inner side cooperating with the profile (51) of the screw (15).

19. A bone fixing system in accordance with claim 18, **characterized in that** the profile (51) of the screw (15) and the counter-profile (53) of the spreading member (17) are made complementary to one another.

20. A bone fixing system in accordance with claim 18 or claim 19, **characterized in that** the profile (15) of the screw (51) and the counter-profile (53) of the spreading member (17) each extend over an axial length which amounts to at least 50% of the length of the spreading member (17).

21. A bone fixing system in accordance with any one of claims 17 to 20, **characterized in that** the profile (51) of the screw (15) is formed by at least one screw section with a varying cross-section which repeats in the axial direction.

22. A bone fixing system in accordance with any one of claims 17 to 21, **characterized in that** the profile (51) of the screw (15) includes a plurality of inclined ramps arranged axially in succession.

23. A bone fixing system in accordance with any one of claims 17 to 22, **characterized in that** the profile (51) of the screw (15) is sawtooth-like.

24. A bone fixing system in accordance with any one of claims 17 to 22, **characterized in that** the profile (51) of the screw (15) is wave-shaped.

## Revendications

1. Système de fixation d'os, en particulier pour la réduction de fractures distales du fémur, comprenant au moins un clou (11), en particulier un clou médullaire du fémur, au moins une vis (15) susceptible d'être passée à travers un perçage transversal (13) ménagé dans le clou (11), en particulier une vis de condyle, et
un dispositif de serrage, qui comprend au moins un élément d'écartement (17) qui entoure au moins localement la vis (15), lequel est déplaçable axialement par rapport à la vis (15) et est susceptible d'être écarté pendant son mouvement de déplacement au moins dans la région du perçage transversal (13),
**caractérisé en ce que** l'élément d'écartement (17) est réalisé en forme de douille.

2. Système de fixation d'os selon la revendication 1,
**caractérisé en ce que** l'élément d'écartement (17) est susceptible d'être écarté par coopération avec au moins un élargissement de section transversale (21) de la vis (15).

3. Système de fixation d'os selon la revendication 2,
**caractérisé en ce que** l'élargissement de section transversale (21) de la vis (15) est prévu sous la forme d'une rampe de montée pour l'élément d'écartement (17), et/ou **en ce que** la vis (15) est réalisée sous forme de cône dans la région de l'élargissement de section transversale (21).

4. Système de fixation d'os selon l'une des revendications précédentes,
**caractérisé en ce que** l'élément d'écartement (17) s'étend sur une longueur axiale de la vis (15) qui va depuis une région au niveau de la tête de vis ou au voisinage de celle-ci jusque dans la région d'un élargissement de section transversale (21) de la vis (15).

5. Système de fixation d'os selon l'une des revendications précédentes,
**caractérisé en ce que** l'épaisseur de paroi de l'élément d'écartement (17) va en se rétrécissant ou converge en pointe en direction axiale dans la région de l'extrémité libre, et/ou la surface de section transversale intérieure libre de l'élément d'écartement (17) dans la région de l'extrémité libre s'élargit en forme de cône en direction axiale.

6. Système de fixation d'os selon l'une des revendications précédentes, **caractérisé en ce que** l'élément d'écartement (17) comprend une pluralité d'éléments de serrage (23, 25) agencés de façon répartie en direction périphérique.

7. Système de fixation d'os selon l'une des revendications précédentes, **caractérisé en ce que** l'élément d'écartement (17) comprend une pluralité d'éléments de serrage (23) séparés les uns des autres simplement par des fentes longitudinales (27), et/ou est réalisé sous forme d'une douille fendue.

8. Système de fixation d'os selon l'une des revendications 1 à 6, **caractérisé en ce que** l'élément d'écartement (17) comprend au moins une paire de languettes de serrage (25) diamétralement opposées l'une par rapport à l'autre, qui recouvrent de préférence chacune une région angulaire périphérique inférieure aux lacunes entre les languettes de serrage (25).

9. Système de fixation d'os selon l'une des revendications précédentes, **caractérisé en ce qu'**un élargissement de section transversale (21) de la vis (15), prévu pour l'élément d'écartement (17), mène depuis une section transversale réduite jusqu'à la section normale de la vis (15).

10. Système de fixation d'os selon l'une des revendications précédentes, **caractérisé en ce que** la section transversale de la vis (15) est réduite exclusivement dans des régions prévues pour des éléments de serrage (23, 25) de l'élément d'écartement (17), et est pourvue à cet effet de préférence d'évidements (49) en forme de gorge ou de goulotte.

11. Système de fixation d'os selon l'une des revendications précédentes, **caractérisé en ce que** le dispositif de serrage est susceptible d'être vissé avec la vis (15).

12. Système de fixation d'os selon l'une des revendications précédentes, **caractérisé en ce que** la vis (15) comporte dans la région de la tête un tronçon fileté (29) destiné au vissage avec le dispositif de serrage.

13. Système de fixation d'os selon l'une des revendications précédentes, **caractérisé en ce qu'**un mouvement de vissage du dispositif de serrage est converti en un mouvement de positionnement axial de l'élément d'écartement (17).

14. Système de fixation d'os selon l'une des revendications précédentes, **caractérisé en ce que** le dispositif de serrage est réalisé en plusieurs pièces et comprend, additionnellement à l'élément d'écartement susceptible d'être enfilé par-dessus la vis (15), un organe de réglage (19) susceptible d'être vissé avec la vis (15), qui est accouplé fermement axialement avec l'élément d'écartement (17), ou susceptible de l'être, en particulier via une liaison à enclenchement ou à encliquetage.

15. Système de fixation d'os selon l'une des revendications précédentes, **caractérisé en ce que** l'organe de réglage (19) et l'élément d'écartement (17) sont susceptibles d'être tournés l'un par rapport à l'autre dans l'état fermement accouplé.

16. Système de fixation d'os selon l'une des revendications précédentes, **caractérisé en ce qu'**il est prévu des moyens de limitation (31, 32) qui fixent une profondeur de pénétration maximum pour la vis (15) dans le perçage (13) du clou (11), dans lequel de préférence le moyen de limitation comprend une butée annulaire (31) sur la paroi intérieure, qui délimite le perçage (13), du clou (11) ainsi qu'un épaulement annulaire (33) réalisé sur la vis (15).

17. Système de fixation d'os selon l'une des revendications précédentes, **caractérisé en ce que** l'élément d'écartement (17) est susceptible d'être écarté, par coopération avec un profilage (51) de la vis (15), simultanément à plusieurs emplacements qui se suivent en direction axiale.

18. Système de fixation d'os selon la revendication 17,
**caractérisé en ce que** l'élément d'écartement (17) présente un profilage antagoniste au niveau de sa face intérieure qui coopère avec le profilage (51) de la vis (15).

19. Système de fixation d'os selon la revendication 18,
**caractérisé en ce que** le profilage (51) de la vis (51) et le profilage antagoniste (53) de l'élément d'écartement (17) sont réalisés complémentaires l'un à l'autre.

20. Système de fixation d'os selon la revendication 18 ou 19,
**caractérisé en ce que** le profilage (15) de la vis (51) et le profilage antagoniste (53) de l'élément d'écartement (17) s'étendent chacun sur une longueur axiale qui est au moins 50 % de la longueur de l'élément d'écartement (17).

21. Système de fixation d'os selon l'une des revendications 17 à 20,
**caractérisé en ce que** le profilage (51) de la vis (15) est formé par au moins un tronçon de vis à section variable qui se répète en direction axiale.

22. Système de fixation d'os selon l'une des revendications 17 à 21,
**caractérisé en ce que** le profilage (51) de la vis (15) comprend une pluralité de rampes de montée agencées axialement les unes derrière les autres.

23. Système de fixation d'os selon l'une des revendications 17 à 22,
**caractérisé en ce que** le profilage (51) de la vis (15) est réalisé à la manière de dents de scie.

24. Système de fixation d'os selon l'une des revendications 17 à 22,
**caractérisé en ce que** le profilage (51) de la vis (15) est réalisé sous forme ondulée.
